(19) Europäisches Patentamt
European Patent Office
Office européen des brevets

(11) Veröffentlichungsnummer: **0 275 893**
**A2**

(12) # EUROPÄISCHE PATENTANMELDUNG

(21) Anmeldenummer: **88100281.0**

(51) Int. Cl.⁴: **C07C 41/48**, C07C 43/315

(22) Anmeldetag: **12.01.88**

Patentanspruch für folgende Vertragsstaat: ES.

(30) Priorität: **17.01.87 DE 3701303**

(43) Veröffentlichungstag der Anmeldung:
**27.07.88 Patentblatt 88/30**

(84) Benannte Vertragsstaaten:
**CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Kleber, Rolf, Dr.**
**Am Trieb 41**
**D-6078 Neu Isenburg(DE)**
Erfinder: **Billenstein, Siegfried, Dr.**
**Samerbergweg 8**
**D-8269 Burgkirchen(DE)**
Erfinder: **Jaeckel, Lothar**
**Kettelerstrasse 88**
**D-6093 Flörsheim am Main(DE)**
Erfinder: **Ignaz, Wimmer**
**Burger Strasse 26**
**D-8261 Winhöring(DE)**

(54) **Polyalkoxiethermischformale.**

(57) Verbindungen der Formel

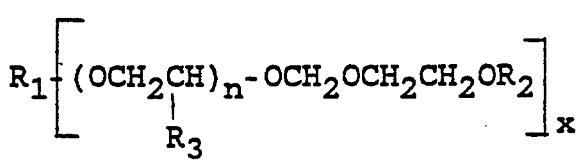

wobei $R_1$ eine Gruppe der Formeln

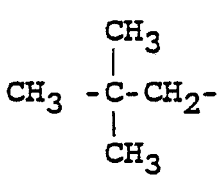 , 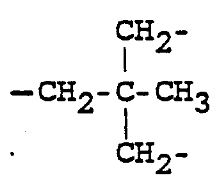 , 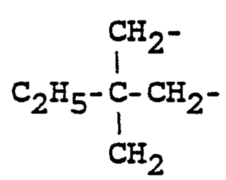 , 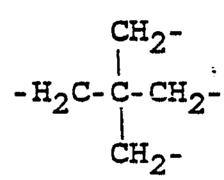

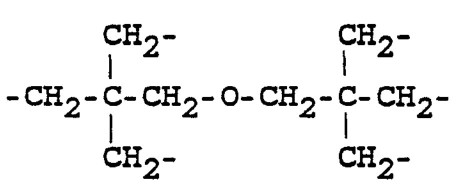 , 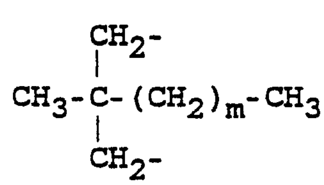

$R_2$ $C_1$-$C_4$-Alkyl, $R_3$ Wasserstoff oder Methyl, n eine Zahl von 5 bis 100, m eine ganze Zahl von 0 bis 4 und x die Anzahl der freien Valenzen des jeweiligen Restes $R_1$ bedeuten, Verfahren zu deren Herstellung und

EP 0 275 893 A2

deren Verwendung als Faserpräparationsmittel.

## Polyalkoxiethermischformale

Aus der DE-C 2 812 443 sind Polyglykolethermischformale der Formel

$R_1-(OC_2H_4)_n-OCH_2OCH_2CH_2OR_2$

bekannt, wobei $R_1$ $C_8$-$C_{22}$-Alkyl, $R_2$ $C_1$-$C_4$-Alkyl und n Zahlen von 3 bis 30 bedeuten. Diese Verbindungen werden als Faserpräparationsmittel eingesetzt und haben sich in der Praxis einen festen Platz erobert. Mit der Zunahme des Friktionstexturierverfahrens auf Polyurethan (PUR)-Scheiben zeigte sich jedoch, daß die in der DT-C 2 812 443 beanspruchten Produkte, trotz niederer Quellneigung für Garne, den Anforderungen der Praxis nicht genügen. Diese Polyglykolethermischformale neigen nämlich zum Anquellen der PUR-Friktionsscheiben, so daß die Standzeiten im Friktionstexturierprozess begrenzt sind. Es besteht daher die Notwendigkeit, Produkte zu entwickeln, welche die niedere Faden/Metallreibung der bekannten Polyglykolethermischformale aufweisen, ohne jedoch PUR-Friktionsscheiben im Praxisbetrieb anzuquellen.

Es wurde nun gefunden, daß diese Bedingungen von solchen Formalen erfüllt werden, die sich von bestimmten mehrfunktionellen Alkoholen ableiten.

Gegenstand der Erfindung sind Verbindungen der Formel 1

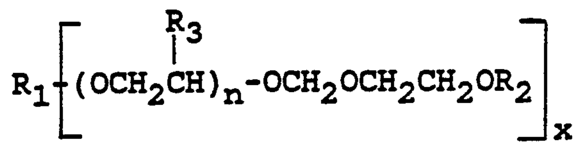 (1)

wobei $R_1$ eine Gruppe der Formeln

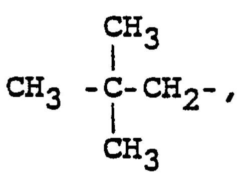 , 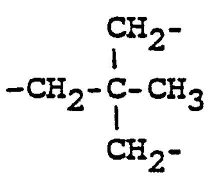 , 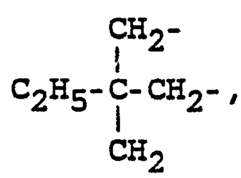 , 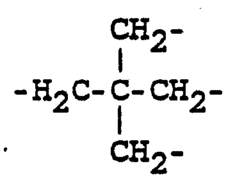

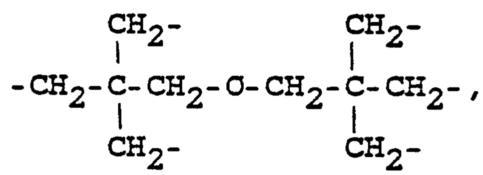 , 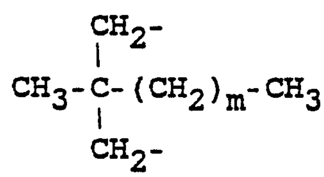

wobei $R_2$ $C_1$-$C_4$-Alkyl, $R_3$ Wasserstoff oder Methyl, n eine Zahl von 5 bis 100, m eine ganze Zahl von 0 bis 4 und x die Anzahl der freien Valenzen des jeweiligen Restes $R_1$ bedeuten.

Die Herstellung dieser Verbindungen erfolgt nach dem in der DE-A 2 523 588 beschriebenen Verfahren, indem man eine Verbindung der Formel (2)

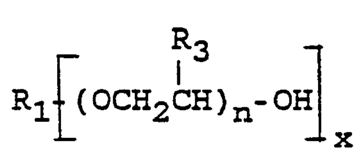 (2)

mit einem Di-alkylglykolformal der Formel (3)

$CH_2(OC_2H_4OR_2)_2$ (3)

in Gegenwart einer starken Säure umsetzt unter gleichzeitiger Entfernung des dabei enstehenden Glykolmonoalkyläthers. Die Verbindungen der Formel (2) werden hergestellt durch Alkoxylierung der Alkohole der Formel $R_1(OH)_x$ mit Ethylen-und/oder Propylenoxid in bekannter Weise, wobei entweder Oxethylate oder Oxpropylate oder Random-bzw. Blockmischpolymerisate vorliegen. Das Molgewicht der Ausgangsoxalkylate wird über die Viskosität bei 50 °C charakterisiert Sehr günstige Verbindungen haben bei 50 °C eine Viskosität von 50-1000 mPa s. Zu hohe Molgewichte führen zum Verölen der PUR-Scheiben durch die

erfindungsgemäßen Verbindungen.

Infolge ihrer unerwarteten niedrigen Faden/Metallreibung, die sich als hohe Gleitwirkung bemerkbar macht, eignen sich die Verbindungen der obigen Formel (1) sehr gut als Gleitmittel. Daneben führen diese Verbindungen, im Gegensatz zu den aus der DE-C 2 812 443 bekannten Verbundungen, nicht zu einem Anquellen der PUR-Scheiben beim Friktionstexturieren.

Die Applikation dieser Mischformale erfolgt nach den üblichen Methoden, beispielsweise durch Pflatschen, Tauchen oder Sprühen. Man kann dabei diese Verbindungen in reiner Form auf die Faser aufbringen oder aber aus verdünnter wäßriger Lösung. Da die Mischformale in Wasser löslich sind bedarf es hierbei, im Gegensatz zu den Mineral-und Esterölen, keiner zusätzlichen Emulgatoren. Diesen Mischformalen kann man auch die bei der Faserpräparation üblichen Komponenten zumischen, wie etwa Antistatika und Fadenschlußmittel. Als Fasertypen, die mit diesen Mischformalen präpariert werden können, kommen sämtliche synthetischen und natürlichen Fasern in Frage, wie etwa Polyester, Polyacrylnitril, Polyamid oder Cellulose. Die Wirksubstanzauflage dieser Mischformale auf der Faser liegt bei Schär-und Webpräparationen bei ca. 0,5 bis 1,5 Gew.-% und bei Texturierpräparationen bei ca. 0,1 bis 1.0 Gew.-%. Sie können auch als wasserfreie Formulierungen für Spulöle oder für Nachavivagen nach dem Färben und nach anderen Verarbeitungsprozessen eingesetzt werden. Dabei beträgt die Wirksubstanzauflage bei Spulölen ca. 1-5 Gew.-% und bei Nachavivagen ca. 0,3-0,8 Gew.-%. Durch ihre Instabilität im sauren pH-Bereich lassen sich diese Präparationen leicht zerstören, indem man nach dem Auswaschen des Präparationsmittels das Abwasser leicht sauer stellt. Die entstehenden Spaltprodukte sind leicht biologisch abbaubar. Teilweise genügt es auch schon Luft durch das Abwasser zu leiten, um die Mischformale zu spalten.

Folgende Mischformale wurden hergestellt analog zu dem in Beispiel 1 der DE-C 2 812 443 angegebenen Verfahren:

a)

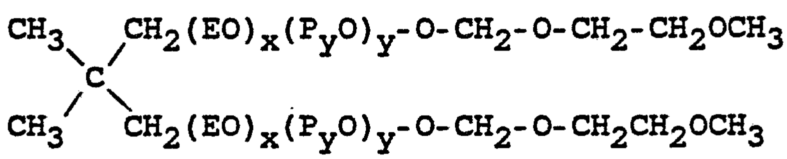

2.2-Dimethylpropandiol-EO/$P_y$)O-Mischoxalkylat-Methylglykol-Mischformal
x + y = random
Verhältnis 4 : 1 Gewichtsteile
Ausgangsviskosität: 50 °C : 200 mPa s

b) $C\,[CH_2\text{-}(EO)_x(P_yO)_y\text{-}O\text{-}CH_2\text{-}O\text{-}CH_2CH_2\text{-}OCH_3]_4$
Pentaerythrit-EO/$P_y$O-Mischoxalkylat-methylglykol-Mischformal
x + y = random
Verhältnis 4 : 1 Gewichtsteile
Ausgangsvikosität des Oxalkylats:
50 °C : 300 mPa s

c) $CH_3\text{-}CH_2\text{-}C\equiv[CH_2(EO)_x(P_yO)_y\text{-}O\text{-}CH_2\text{-}O\text{-}CH_2CH_2\text{-}OCH_3]_3$
Trimethylolpropan-EO/$P_y$O-Mischoxalkylat-methylglykol-Mischformal
x + y = random
Verhältnis 1 : 4 Gewichtsteile
Ausgangsviskosität des Oxalkylats:
50 °C : 100 mPa s.

Zum Vergleich wurden die Verbindungen

d) $C_{12/145}(EO)\text{-}O\text{-}CH_2\text{-}O\text{-}CH_2CH_2\text{-}O\text{-}CH_3$ (DE-C 2 812 443)

e) $C_{12/14}\text{-}(EO)_5\text{-}O\text{-}CH_2\text{-}O\text{-}n\text{-}C_4H_9$ (DE-A 2 523 588)

f) $C_{12/14}(EO)_5\text{-}O\text{-}C_4H_9$ (US-A 3 997 450) hergestellt.

In diese Produkte wurden PUR-Scheiben eingelegt, so daß die Scheiben völlig bedeckt waren und im verschlossenen Glasbehälter 7 Tage auf 90 °C erwärmt.

Nach dieser Zeit wurden die Scheiben wie folgt visuell beurteilt:

| Produkt: | PUR-Scheibe |
|---|---|
| a: | unverändert |
| b: | unverändert |
| c: | unverändert |
| d: Vergleich | zerstört |
| e: Vergleich | zerstört |
| f: Vergleich | zerstört |

Ähnliche Effekte werden erhalten für die erfindungsgemäße Verbindung
$TME(EO)_x(P_yO)_y$-O-$CH_2$-O-$CH_2$-$CH_2$-O-$nC_4H_9$
x + y = Block
x + y = 1 : 1
Viskosität bei 50 °C : 400 mPa s
TME = Trimetholylethyl

## Ansprüche

1. Verbindungen der Formel

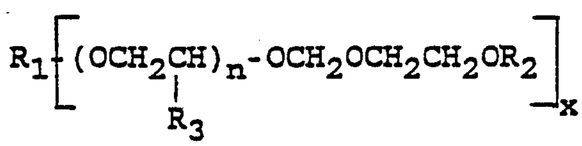

wobei $R_1$ eine Gruppe der Formeln

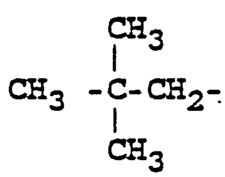,
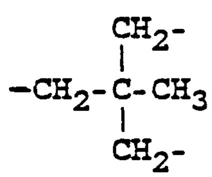,
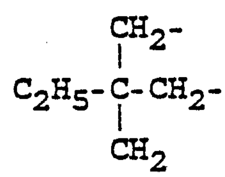,
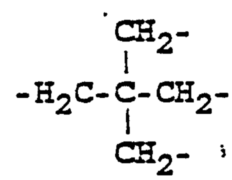

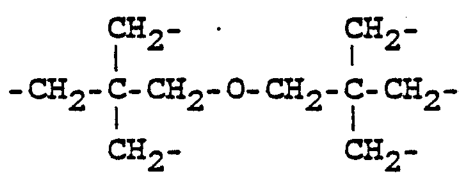,
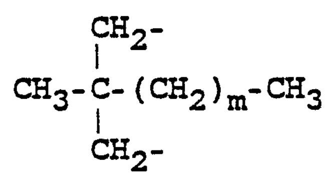

$R_2$ $C_1$-$C_4$-Alkyl, $R_3$ Wasserstoff oder Methyl, n eine Zahl von 5 bis 100, m eine ganze Zahl von 0 bis 4 und x die Anzahl der freien Valenzen des jeweiligen Restes $R_1$ bedeuten.

2. Verfahren zur Herstellung der Verbindung nach Anspruch 1, dadurch gekennzeichnet, daß man eine Verbindung der Formel

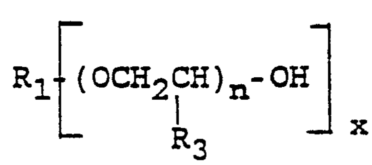

mit einem Di-alkylglykolformal der Formel
$CH_2(OC_2H_4OR_2)_2$

in Gegenwart einer starken Säure umsetzt unter gleichzeitigen Entfernung des dabei entstehenden Glykolmonoalkyläthers.

3. Verwendung der Verbindungen nach Anspruch 1 als Faserpräparationsmittel.

Patentansprüche fur den folgenden Vertragsstaat: ES

1. Verfahren zur Herstellung von Verbindungen der Formel

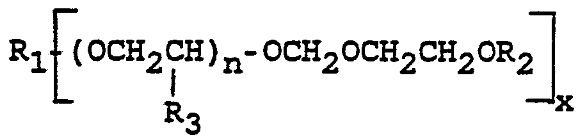

wobei $R_1$ eine Gruppe der Formeln

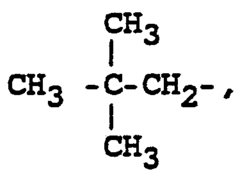,
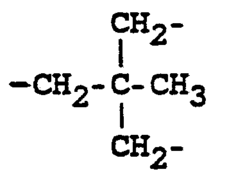,
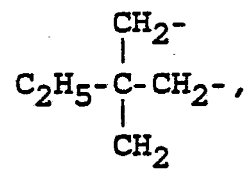,
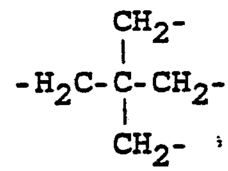;

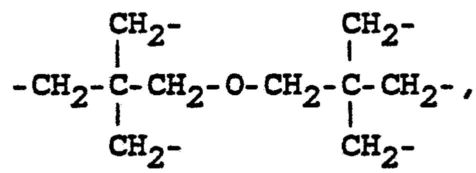,
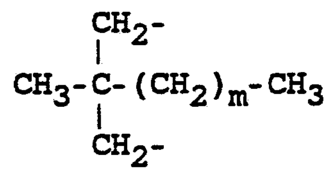

$R_2$ $C_1$-$C_4$-Alkyl, $R_3$ Wasserstoff oder Methyl, n eine Zahl von 5 bis 100, m eine ganze Zahl von 2 bis 4 und x die Anzahl der freien Valenzen des jeweiligen Restes $R_1$ bedeuten, dadurch gekennzeichnet, daß man eine Verbindung der Formel

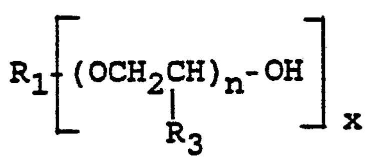

mit einem Di-alkylglykolformal der Formel
$CH_2(OC_2H_4OR_2)_2$
in Gegenwart einer starken Säure umsetzt unter gleichzeitigen Entfernung des dabei entstehenden Glykolmonoalkyläthers.

2. Verfahren zur Präparation von Textilfasern, dadurch gekennzeichnet, daß man auf die Textilfasern 0,1 bis 1,5 Gew.-% der Verbindungen nach Anspruch 1 aufbringt.